# EUROPEAN PATENT APPLICATION

(11) **EP 3 533 477 A1**
(43) Date of publication of application: **04.09.2019**
(21) Application number: 16920403.9
(22) Date of filing: 05.12.2016
(51) Int. Cl.: A61L 31/02, C22C 23/04, B21C 23/00

(54) **BIODEGRADABLE MAGNESIUM ALLOY AND MANUFACTURING METHOD THEREFOR**

(30) Priority: 31.10.2016 KR 20160143642
(71) Applicant: U & I Corporation, Uijeongbu-si Gyeonggi-do 11781 (KR)
(72) Inventor: KIM, Yu Chan, Seoul 02792 (KR); SEOK, Hyun Kwang, Seoul 02792 (KR); HAN, Hyung-Seop, Seoul 02792 (KR); BYUN, Ji Young, Seoul 02792 (KR); JEON, Hojeong, Seoul 02792 (KR); OK, Myoung-Ryul, Seoul 02792 (KR); PARK, Jimin, Seoul 02792 (KR); YANG, Seok-Jo, Seoul 02792 (KR)
(74) Representative: Shearman, James Ward
(86) International application number: PCT/KR2016/014172
(87) International publication number: WO 2018/079923

(57) **Abstract**

The present invention relates to a biodegradable magnesium alloy comprising x wt% of calcium, y wt% of zinc, and the balance of magnesium and inevitable impurities, wherein x and y have a range corresponding to a region in which the lower part of the trajectory of mathematical formula 1 (y = 44.894x² - 25.123x + 5.192) and the upper part of the trajectory of mathematical formula 2 (y = - 10.618x² + 7.8784x + 0.1637) overlap in the x-y plane, and a zinc compound phase contains 90 wt% or more of Ca₂Mg₆Zn₃ on the basis of the total weight of the zinc compound phase.

## Description

### TECHNICAL FIELD

The present invention relates to a magnesium alloy and a manufacturing method therefor, and more particularly, to a biodegradable magnesium alloy and a manufacturing method therefor.

### BACKGROUND ART

A magnesium alloy is easy to mold, but there is a disadvantage of having poor corrosion resistance and strength. To improve the corrosion resistance and strength of the magnesium alloy, studies for appropriately changing a composition of the magnesium alloy had been conducted. As the result through the studies, it was found that the mechanical strength was improved as an amount of an additive was increased. Meanwhile, as the amount of an additive increases, various phases are formed, and as electric potential differences therebetween are large, a galvanic circuit, which promotes a corrosion rate, is easily formed. Accordingly, there has been demand for a study on a magnesium alloy having excellent corrosion resistance, strength, and elongation while being controlling corrosion characteristics.

Related prior arts include Korean Patent Publication No. 20120062243 (published on June 14, 2012, entitled "DEGRADABILITY VELOCITY CONTROL METHOD OF BIODEGRADABLE MAGNESIUM AND BIODEGRADABLE MAGNESIUM USING THEREOF").

### DISCLOSURE OF THE INVENTION

### TECHNICAL PROBLEM

The purpose of the present invention is to provide: a biodegradable magnesium alloy having excellent corrosion resistance, strength, and elongation; and a manufacturing method therefor. However, these problems are exemplary and the scope of the present invention is not limited thereto.

### TECHNICAL SOLUTION

There is provided a biodegradable magnesium alloy according to an aspect of the present invention. The biodegradable magnesium alloy contains 5 wt% or less (more than 0) of zinc, 0.35 wt% or less (more than 0) of calcium, and the balance of magnesium and inevitable impurities, wherein a microstructure of the magnesium alloy includes an α-Mg phase matrix and a zinc compound phase which is dispersed and precipitated in the form of particles in the matrix, and the zinc compound phase contains 90 wt% or more of Ca₂Mg₆Zn₃ on the basis of the total weight of the zinc compound phase.

In the biodegradable magnesium alloy, the microstructure of the magnesium alloy may be formed only of both an α-Mg phase matrix and a Ca₂Mg₆Zn₃ phase which is dispersed and precipitated in the matrix.

In the biodegradable magnesium alloy, the calcium may be contained in an amount of 0.05-0.35 wt% in the alloy.

There is provided a biodegradable magnesium alloy according to another aspect of the present invention. The biodegradable magnesium alloy is formed of x wt% of calcium, y wt% of zinc, and the balance of magnesium and inevitable impurities, wherein x and y have a range corresponding to a region in which the lower part of the trajectory of mathematical formula 1 (y = 44.894x² - 25.123x + 5.192) and the upper part of the trajectory of mathematical formula 2 (y = -10.618x² + 7.8784x + 0.1637) overlap in the x-y plane, and a zinc compound phase contains 90 wt% or more of Ca₂Mg₆Zn₃ on the basis of the total weight of the zinc compound phase.

There is provided a biodegradable magnesium alloy according to another aspect of the present invention. The biodegradable magnesium alloy contains 5 wt% or less (more than 0) of zinc, x wt% or less (more than 0) of calcium, and the balance of magnesium and inevitable impurities, wherein x is a maximum allowable amount of calcium permitting the existence of a temperature section in which a structure formed only of an α-Mg single-phase is phase-transformed into a structure formed of an α-Mg phase matrix and particles, which are dispersed and precipitated in the matrix and formed only of a Ca₂Mg₆Zn₃ phase, in a process of naturally cooling the molten biodegradable magnesium alloy.

There is provided a biodegradable magnesium alloy according to another aspect of the present invention. The biodegradable magnesium alloy contains 5 wt% or less (more than 0) of zinc and the balance of magnesium and inevitable impurities, wherein a microstructure of the magnesium alloy includes an α-Mg phase matrix and a zinc compound phase which is dispersed and precipitated in the form of particles in the matrix.

There is provided a manufacturing method for a biodegradable magnesium alloy according to still another aspect of the present invention. The method includes: casting a molten metal having a composition of the described magnesium alloy; performing solution treatment of the cast magnesium alloy in a temperature range of 300-400°C; and performing hot extrusion in a temperature range of 300-400°C after performing the solution treatment.

In the manufacturing method for a biodegradable magnesium alloy, the method may further include performing artificial aging treatment for 17-19 hours after performing the hot extrusion.

In addition to the above-described aspects, there is provided an implant according to another aspect of the present invention. The implant includes the described biodegradable magnesium alloy, and is also used for orthopedic surgery, dentistry, plastic surgery, or vascular surgery.

### ADVANTAGEOUS EFFECTS

According to an embodiment of the present invention as described above, a biodegradable magnesium alloy having excellent corrosion resistance, strength, and elongation and a manufacturing method therefor may be achieved. However, the effects are exemplary, and the scope of the present invention is not limited thereto.

### BRIEF DESCRIPTION OF THE DRAWINGS

FIG. 1 is a diagram showing a portion of a constitutional diagram of an Mg-1Zn-xCa ternary alloy which is formed of 1 wt% of zinc and the balance of magnesium while varying a content of calcium.
FIG. 2 is a diagram showing a portion of a constitutional diagram of an Mg-1.5Zn-xCa ternary alloy which is formed of 1.5 wt% of zinc and the balance of magnesium while varying a content of calcium.
FIG. 3 is a diagram showing a portion of a constitutional diagram of an Mg-1.6Zn-xCa ternary alloy which is formed of 1.6 wt% of zinc and the balance of magnesium while varying a content of calcium.
FIG. 4 is a diagram showing a portion of a constitutional diagram of an Mg-1.7Zn-xCa ternary alloy which is formed of 1.7 wt% of zinc and the balance of magnesium while varying a content of calcium.
FIG. 5 is a diagram showing a portion of a constitutional diagram of an Mg-1.8Zn-xCa ternary alloy which is formed of 1.8 wt% of zinc and the balance of magnesium while varying a content of calcium.
FIG. 6 is a diagram showing a portion of a constitutional diagram of an Mg-2Zn-xCa ternary alloy which is formed of 2.0 wt% of zinc and the balance of magnesium while varying a content of calcium.
FIG. 7 is a diagram showing a portion of a constitutional diagram of an Mg-3Zn-xCa ternary alloy which is formed of 3.0 wt% of zinc and the balance of magnesium while varying a content of calcium.
FIG. 8 is a diagram showing a portion of a constitutional diagram of an Mg-4Zn-xCa ternary alloy which is formed of 4.0 wt% of zinc and the balance of magnesium while varying a content of calcium.
FIG. 9 is a diagram showing a portion of a constitutional diagram of an Mg-5Zn-xCa ternary alloy which is formed of 5.0 wt% of zinc and the balance of magnesium while varying a content of calcium.
FIGS. 10 and 11 are graphs showing mathematical formulae 1 and 2 which define composition ranges of calcium and zinc.
FIG. 12 is a graph showing a composition range of calcium and zinc in a biodegradable magnesium alloy according to an embodiment of the present invention.
FIG. 13 is a graph showing comparative analysis of corrosion characteristics of biodegradable magnesium alloys according to other Experimental Examples of the present invention.

### MODE FOR CARRYING OUT THE INVENTION

Hereinafter, the present invention will be described in detail by explaining preferred embodiments of the invention with reference to the attached drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. It should be noted that elements shown in the accompanying drawings may be scaled up or down for convenience in description. Like reference numerals in the drawings denote like elements.

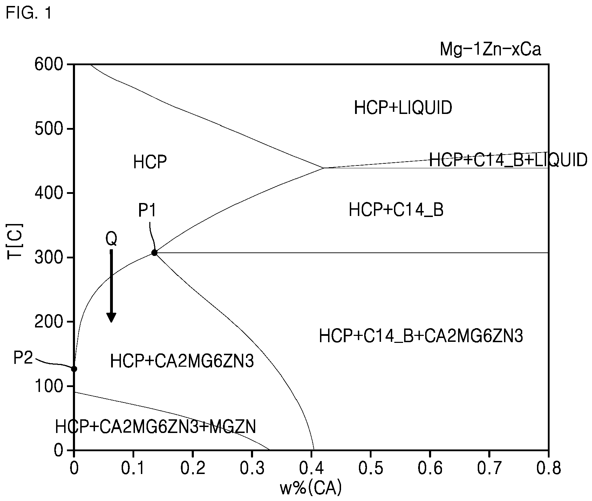

FIG. 1 is a diagram showing a portion of a constitutional diagram of an Mg-1Zn-xCa ternary alloy which is formed of 1 wt% of zinc and the balance of magnesium while varying a content of calcium. Referring to FIG. 1, a boundary line connecting P1 and P2 is a boundary at which a structure formed only of an α-Mg single-phase (corresponding to a region denoted by 'HCP' in FIG. 1) is transformed into a structure formed of an α-Mg phase matrix and particles, which are dispersed and precipitated in the matrix and formed only of a Ca₂Mg₆Zn₃ phase (corresponding to the region denoted by 'HCP+CA2MG6ZN3' in FIG. 1). That is, when high-temperature Mg (HCP) is cooled (Q) by passing through the boundary line connecting P1 and P2, Ca₂Mg₆Zn₃ is only precipitated in the magnesium matrix. Accordingly, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.131. The inventors of the present invention confirmed that corrosion resistance and strength were relatively excellent when the dispersed and precipitated particles in the α-Mg phase matrix were formed only of the Ca₂Mg₆Zn₃ single-phase.

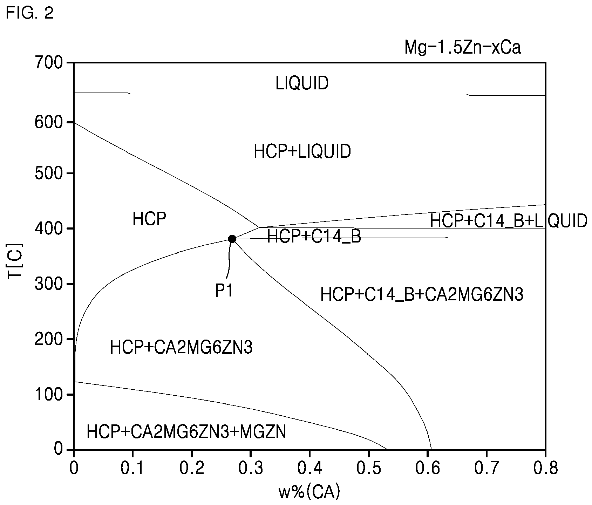

FIG. 2 is a diagram showing a portion of a constitutional diagram of an Mg-1.5Zn-xCa ternary alloy which is formed of 1.5 wt% of zinc and the balance of magnesium while varying a content of calcium. Referring to FIG. 2, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.268. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

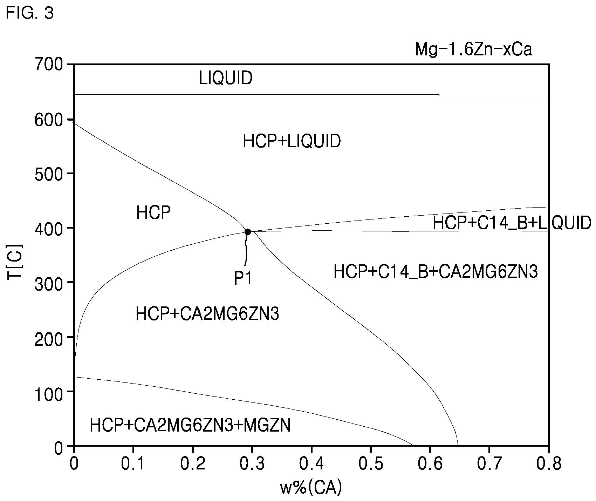

FIG. 3 is a diagram showing a portion of a constitutional diagram of an Mg-1.6Zn-xCa ternary alloy which is formed of 1.6 wt% of zinc and the balance of magnesium while varying a content of calcium. Referring to FIG. 3, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.2907. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

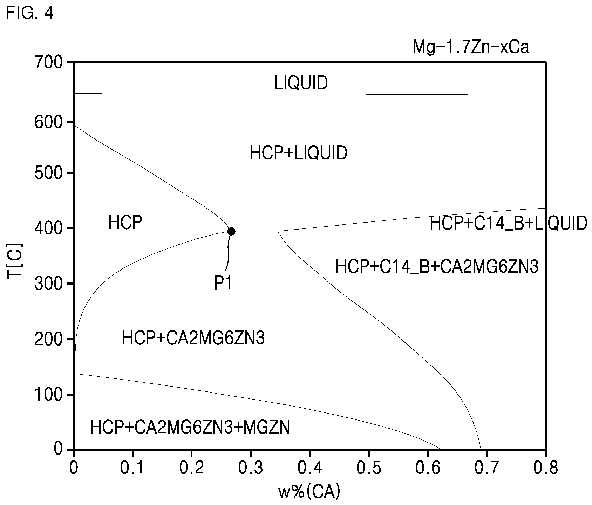

FIG. 4 is a diagram showing a portion of a constitutional diagram of an Mg-1.7Zn-xCa ternary alloy which is formed of 1.7 wt% of zinc and the balance of magnesium while varying a content of calcium. Referring to FIG. 4, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.2661. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

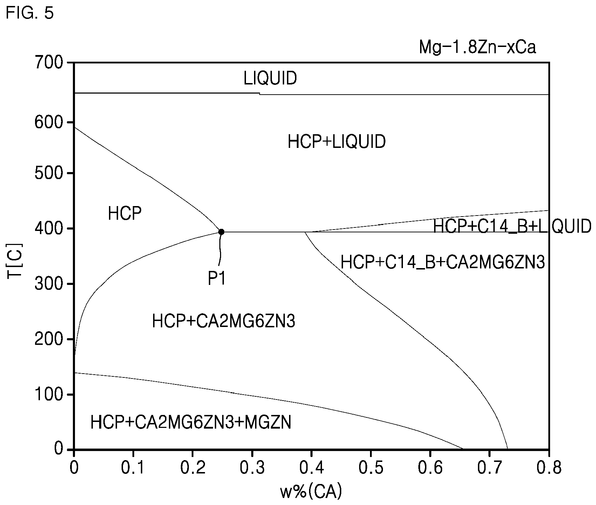

FIG. 5 is a diagram showing a portion of a constitutional diagram of an Mg-1.8Zn-xCa ternary alloy which is formed of 1.8 wt% of zinc and the balance of magnesium while varying a content of calcium. Referring to FIG. 5, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.2435. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

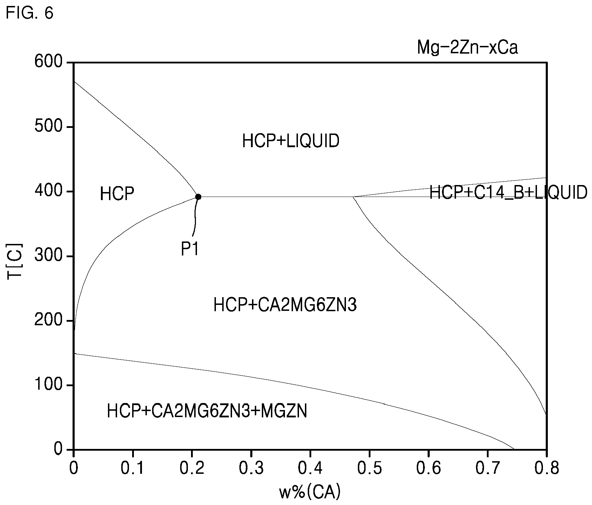

FIG. 6 is a diagram showing a portion of a constitutional diagram of an Mg-2Zn-xCa ternary alloy which is formed of 2.0 wt% of zinc and the balance of magnesium while varying a content of calcium. Referring to FIG. 6, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.2036. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

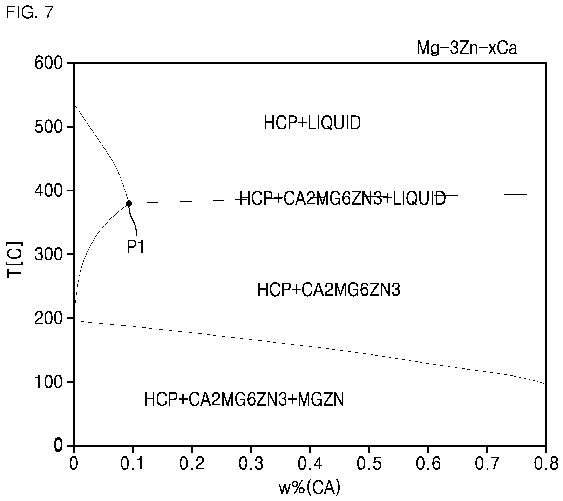

FIG. 7 is a diagram showing a portion of a constitutional diagram of an Mg-3Zn-xCa ternary alloy which is formed of 3.0 wt% of zinc and the balance of magnesium while varying a content of calcium. Referring to FIG. 7, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.105. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

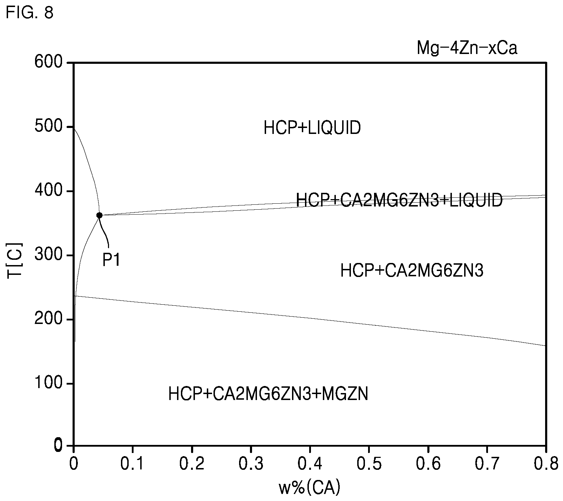

FIG. 8 is a diagram showing a portion of a constitutional diagram of an Mg-4Zn-xCa ternary alloy which is formed of 4.0 wt% of zinc and the balance of magnesium while varying a content of calcium. Referring to FIG. 8, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.04015. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

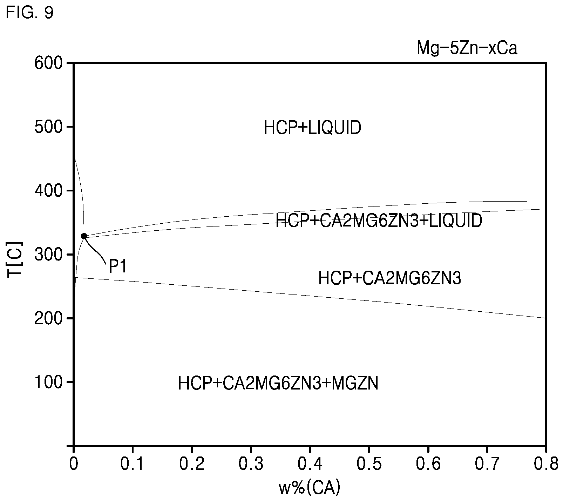

FIG. 9 is a diagram showing a portion of a constitutional diagram of an Mg-5Zn-xCa ternary alloy which is formed of 5.0 wt% of zinc and the balance of magnesium while varying a content of calcium. Referring to FIG. 9, it may be understood that P1 wt% of calcium is a maximum allowable amount of calcium permitting the existence of a temperature section in which a structure formed only of an α-Mg single-phase is phase-transformed into a structure formed of an α-Mg phase matrix and Ca₂Mg₆Zn₃ which is only dispersed and precipitated in the matrix, in a process of naturally cooling the molten magnesium alloy. In the alloy of the embodiment, such P1 has a value of 0.0167. In the embodiment, it was also confirmed that corrosion resistance and strength were relatively excellent when the dispersed and precipitated particles in the α-Mg phase matrix were formed only of the Ca₂Mg₆Zn₃ single-phase. The rest of the description is replaced with the description in FIG. 1.

The magnesium alloy according to the described embodiments may be easily applied to an implant member for orthopedic surgery, dentistry, plastic surgery, or vascular surgery because a biodegradation rate thereof may be easily controlled and the strength and corrosion resistance thereof are excellent. Such a magnesium alloy includes an α-Mg phase matrix and a zinc compound phase which is dispersed and precipitated in the form of particles in the matrix. The zinc compound phase may contain 90 wt% or more of Ca₂Mg₆Zn₃ on the basis of the total weight of the zinc compound phase. The zinc compound phase may particularly contain 98 wt% or more of Ca₂Mg₆Zn₃ on the basis of the total weight of the zinc compound phase, and more particularly, the zinc compound phase which is dispersed and precipitated in the form of particles in the matrix may be formed only of Ca₂Mg₆Zn₃.

The inventors of the present invention confirmed that a biodegradable magnesium alloy, which may be applied to an implant member for orthopedic surgery, dentistry, plastic surgery, or vascular surgery because having excellent the corrosion resistance and strength, may be achieved when the magnesium alloy has a composition in a predetermined range bounded by the values derived from FIGS. 1 to 9. Hereinafter, the composition range will be described.

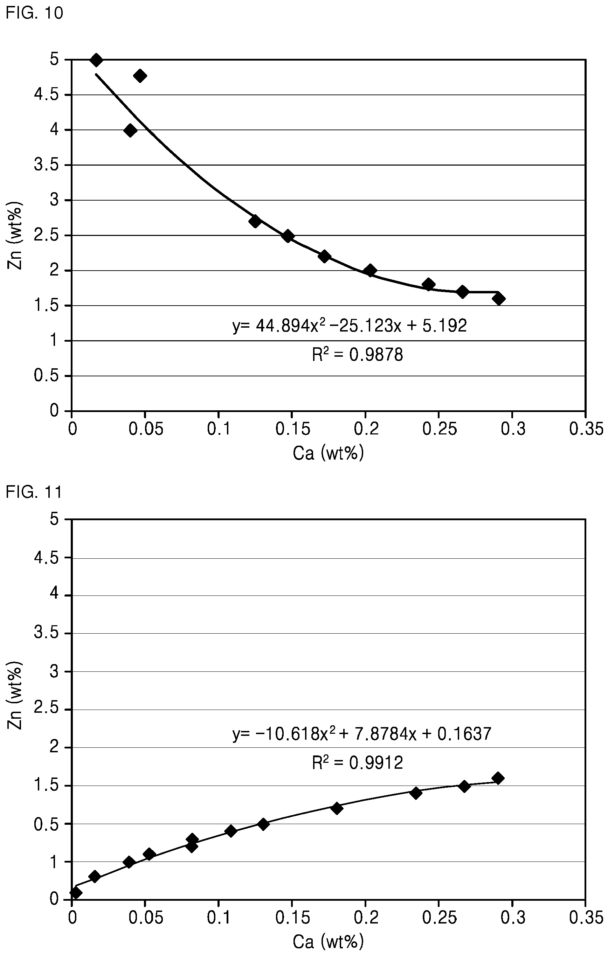

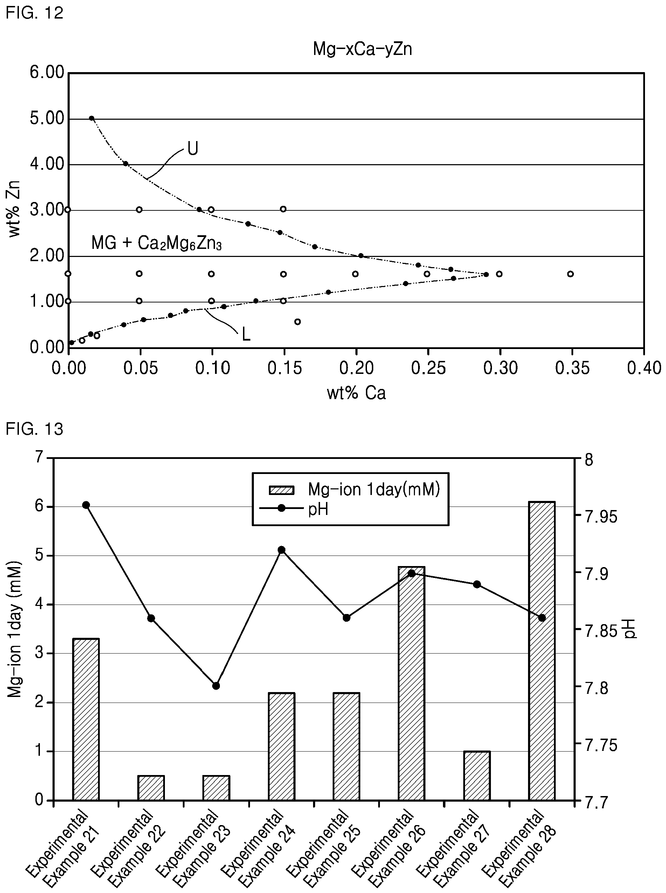

FIGS. 10 and 11 are graphs showing mathematical formulae 1 and 2 which define composition ranges of calcium and zinc, and FIG. 12 is a graph showing a composition range of calcium and zinc in a biodegradable magnesium alloy according to an embodiment of the present invention.

Referring to FIG. 10, a trajectory of mathematical formula 1 (y = 44.894x² - 25.123x + 5.192) defining the composition range of calcium and zinc is shown. That is, it may be confirmed that a maximum allowable amount of calcium permitting the existence of a temperature section, in which a structure formed only of an α-Mg single-phase is phase-transformed into a structure formed of an α-Mg phase matrix and Ca₂Mg₆Zn₃ which is only dispersed and precipitated in the matrix while fixing a zinc content of 1.6 wt% or more in a process of naturally cooling the molten magnesium alloy, is derived, and thereafter, when the maximum allowable amount of calcium as a x-coordinate value and the zinc content as a y-coordinate value are plotted in the x-y plane, the y value of the zinc content and the x value of the maximum allowable amount of calcium have functional relation to mathematical formula 1 (y = 44.894x² - 25.123x + 5.192). The coordinate values (x, y) provided to derive mathematical formula 1 are determined by the manner described in FIGS. 1 to 9, and the values are, for example, (0.0167, 5), (0.04015, 4), (0.125, 2.7) (0.1474, 2.5), (0.1719, 2.2), (0.2036, 2), (0.2435, 1.8), (0.2661, 1.7), and (0.2907, 1.6).

Referring to FIG. 11, a trajectory of mathematical formula 2 (y = -10.618x² + 7.8784x + 0.1637) defining the composition range of calcium and zinc is shown. That is, it may be confirmed that a maximum allowable amount of calcium permitting the existence of a temperature section, in which a structure formed only of an α-Mg single-phase is phase-transformed into a structure formed of an α-Mg phase matrix and Ca₂Mg₆Zn₃ which is only dispersed and precipitated in the matrix while fixing a zinc content of 1.6 wt% or more in a process of naturally cooling the molten magnesium alloy, is derived, and thereafter, when the maximum allowable amount of calcium as a x-coordinate value and the zinc content as a y-coordinate value are plotted in the x-y plane, the y value of the zinc content and the x value of the maximum allowable amount of calcium have functional relation to mathematical formula 2 (y = -10.618x² + 7.8784x + 0.1637). The coordinate values of (x, y) provided to derive mathematical formula 2 are determined by the manner described in FIGS. 1 to 9, and the values are, for example, (0.00269, 0.1), (0.01573, 0.3), (0.0389, 0.5), (0.05293. 0.6), (0.128, 0.7), (0.1086, 0.9), (0.131, 1.0), (0.1809, 1.2), (0.2351, 1.4), (0.268, 1.5), and (0.2907, 1.6).

Referring to FIG. 12, the biodegradable magnesium alloy according to an embodiment of the present invention is formed of x wt% of calcium, y wt% of zinc, and the balance of magnesium and inevitable impurities, wherein x and y may have a range corresponding to a region in which the lower part of the trajectory of mathematical formula 1 (y = 44.894x² - 25.123x + 5.192) and the upper part of the trajectory of mathematical formula 2 (y = -10.618x² + 7.8784x + 0.1637) overlap in the x-y plane. In the embodiment, a zinc compound phase may contain 90 wt% or more of Ca₂Mg₆Zn₃ on the basis of the total weight of the zinc compound phase, thereby achieving a biodegradable magnesium alloy having excellent corrosion resistance and strength.

The points shown in FIG. 12 are composition examples of the alloys which have been experimented to confirm the embodiment, and the biodegradable magnesium alloy having a calcium and zinc composition corresponding to a first region, in which the lower part of the trajectory (U) of mathematical formula 1 and the upper part of the trajectory (L) of mathematical formula 2 overlap in the x-y plane, has a single precipitated phase, whereas the biodegradable magnesium alloy having a calcium and zinc composition corresponding to a region outside the first region has two or more precipitated phases. The inventors of the present invention confirmed that the biodegradable magnesium alloy had the more excellent corrosion resistance and strength when having the single precipitated phase than having the two or more precipitated phases.

Meanwhile, according to another embodiment of the present invention, it was confirmed that a molten metal having the described composition was cast and the cast magnesium alloy was additionally heat-treated to increase the strength and elongation thereof. For example, the strength and elongation of the magnesium alloy were increased by performing: cast of a molten metal having the described composition and solution treatment of the cast magnesium alloy in a temperature range of 300-400°C; and hot extrusion in a temperature range of 300-400°C after performing the solution treatment, and thereafter, artificial aging treatment for 17-19 hours. However, performing the artificial aging in the technical concept of the present invention is not essential and may be omitted selectively. Furthermore, even in the step for performing the artificial aging treatment, the strength and elongation were significantly increased when performing for 17-19 hours, but the technical concept of the present invention is not limited to the described specific time.

Hereinafter, the strength and elongation characteristics of the biodegradable magnesium alloy according to Experimental Examples of the present invention are compared and analyzed.

**[Table 1]**

| Experimental Example | Composition (wt%) | | Non-heat treatment | | | Heat treatment | | |
|---|---|---|---|---|---|---|---|---|
| | | | UTS (MPa) | Elongation (%) | Parameter | UTS (MPa) | Elongation (%) | Parameter |
| 1 | Mg | Balanced | 277.49 | 15.68 | 43.52 | 282.80 | 17.81 | 50.36 |
| | Ca | 0 | | | | | | |
| | Zn | 1.6 | | | | | | |
| 2 | Mg | Balanced | 253.90 | 26.12 | 66.31 | 255.25 | 29.90 | 76.32 |
| | Ca | 0.05 | | | | | | |
| | Zn | 1.6 | | | | | | |
| 3 | Mg | Balanced | 265.92 | 25.85 | 68.74 | 231.66 | 27.90 | 64.63 |
| | Ca | 0.1 | | | | | | |
| | Zn | 1.6 | | | | | | |
| 4 | Mg | Balanced | 250.88 | 27.57 | 69.16 | 257.91 | 25.88 | 66.76 |
| | Ca | 0.15 | | | | | | |
| | Zn | 1.6 | | | | | | |
| 5 | Mg | Balanced | 260.67 | 26.35 | 68.69 | 276.91 | 23.99 | 66.44 |
| | Ca | 0.2 | | | | | | |
| | Zn | 1.6 | | | | | | |
| 7 | Mg | Balanced | 254.35 | 26.46 | 67.30 | 282.03 | 21.82 | 61.55 |
| | Ca | 0.3 | | | | | | |
| | Zn | 1.6 | | | | | | |
| 9 | Mg | Balanced | 217.41 | 17.66 | 38.39 | 287.58 | 20.55 | 59.09 |
| | Ca | 0.01 | | | | | | |
| | Zn | 0.15 | | | | | | |
| 10 | Mg | Balanced | 219.43 | 20.77 | 45.58 | 249.02 | 21.08 | 52.49 |
| | Ca | 0 | | | | | | |
| | Zn | 1 | | | | | | |
| 11 | Mg | Balanced | 225.40 | 18.75 | 42.25 | 213.06 | 20.03 | 42.68 |
| | Ca | 0.02 | | | | | | |
| | Zn | 0.25 | | | | | | |
| 12 | Mg | Balanced | 247.00 | 31.90 | 78.79 | 259.85 | 29.63 | 77.00 |
| | Ca | 0.05 | | | | | | |
| | Zn | 1 | | | | | | |
| 13 | Mg | Balanced | 260.56 | 24.69 | 64.34 | 274.20 | 26.49 | 72.63 |
| | Ca | 0.05 | | | | | | |
| | Zn | 3 | | | | | | |
| 14 | Mg | Balanced | 250.16 | 29.95 | 74.93 | 254.54 | 27.06 | 68.87 |
| | Ca | 0.1 | | | | | | |
| | Zn | 1 | | | | | | |
| 19 | Mg | Balanced | 269.56 | 24.71 | 66.62 | 280.68 | 27.33 | 76.72 |
| | Ca | 0.16 | | | | | | |
| | Zn | 0.55 | | | | | | |

In Table 1, the heat treatment includes T4 heat treatment or T6 heat treatment. For example, the T4 heat treatment may include a condition of quenching after the solution treatment at a temperature of about 400°C for 6 hours. The T6 heat treatment may also include a condition of quenching after the solution treatment at a temperature of about 300-400°C for 6 hours, and then, artificial aging at a temperature of 200°C for 17-19 hours.

In Table 1, UTS refers to the ultimate tensile strength, and it means a value that the maximum load of the material, which can withstand when it is tensed to be cut, is divided by the cross-sectional area of the material, and the elongation means a percentage that the material elongates in the tensile test. Generally, the maximum tensile strength and the elongation have a contrast tendency from each other, and accordingly, in Table 1, the physical properties of the biodegradable magnesium alloy were evaluated by introducing a first parameter represented by dividing the product of the maximum tensile strength and the elongation by 100.

Referring to Table 1, the biodegradable magnesium alloy containing 5 wt% or less (more than 0) of zinc, 0.05-0.35 wt% of calcium, and the balance of magnesium and inevitable impurities (Experimental Examples 2, 3, 4, 5, 7, 12, 13, and 14) had the first parameter value exceeding 50 even when the heat treatment was not performed, and had the first parameter value exceeding 60 when the heat treatment was performed. On the contrary, referring to Experimental Examples 1, 9, 10, 11, and 18, respectively, the magnesium alloy, in which calcium is not present or only very small amount of calcium of less than 0.05 wt% is present, had the first parameter value of less than 50 when the heat treatment was not performed, and had the first parameter value of less than 60 even when the heat treatment was performed.

In the biodegradable magnesium alloys according to Experimental Examples 2, 3, 4, 5, 7, 12, 13, and 14 described above, it was confirmed that each of the magnesium alloys had the calcium and zinc composition corresponding to the first region, in which the lower part of the trajectory (U) of mathematical formula 1 shown in FIG. 12 and the upper part of the trajectory (L) of mathematical formula 2 shown in FIG. 12 overlap in the x-y plane. Accordingly, it may be confirmed that the biodegradable magnesium alloy had the more excellent corrosion resistance and strength when the magnesium alloy matrix had the single precipitated phase (Ca₂Mg₆Zn₃) than had two or more precipitated phases.

Hereinafter, corrosion characteristics of the biodegradable magnesium alloys according to other Experimental Examples of the present invention were compared and analyzed. FIG. 13 is a graph showing comparative analysis of corrosion characteristics of the biodegradable magnesium alloys according to other Experimental Examples of the present invention.

First of all, referring to Table 2, the alloys in Experimental Examples 21, 22, 24, 25, and 27 contain the compositions of Mg-0.56Zn-0.037Ca, Mg-0.99Zn-0.029Ca, Mg-1.63Zn-0.059 Ca, Mg-1.61Zn-0.14Ca, and Mg-2.94Zn-0.00175Ca, respectively, and each of the calcium and zinc compositions has a range corresponding to the region in which the lower part of the trajectory of mathematical formula 1 and the upper part of the trajectory of mathematical formula 2 overlap in the x-y plane. Among Experimental Examples above, the composition in Experimental Example 21 is located in a region very close to the trajectory of mathematical formula 2. On the other hand, the alloys in Experimental Examples 26 and 28 contain the compositions of Mg-1.7Zn-0.3Ca and Mg-3Zn-0.2Ca, respectively, and each of the calcium and zinc compositions has a range corresponding to outside of the region in which the lower part of the trajectory of mathematical formula 1 and the upper part of the trajectory of mathematical formula 2 overlap in the x-y plane.

**[Table 2]**

| Experimental Example | Ca composition | Zn composition | Mg composition |
|---|---|---|---|
| 21 | 0.037 | 0.56 | Balanced |
| 22 | 0.029 | 0.99 | Balanced |
| 23 | 0 | 1.65 | Balanced |
| 24 | 0.059 | 1.63 | Balanced |
| 25 | 0.14 | 1.61 | Balanced |
| 26 | 0.3 | 1.7 | Balanced |
| 27 | 0.00175 | 2.94 | Balanced |
| 28 | 0.2 | 3 | Balanced |

The vertical axis in FIG. 13 indicates a magnesium elution amount in the corrosion test, and the amount may be understood as an index indicating a corrosion rate. Accordingly, it may be confirmed that the corrosion rates of Experimental Examples 26 and 28 are much faster than those of other Experimental Examples. In addition, it may be confirmed that the corrosion rate of Experimental Example 21, in which the composition is adjacent to the trajectory defined by mathematical formulae 1 and 2, is a moderate rate, and the corrosion rates of Experimental Examples 22, 23, 24, 25, and 27 are relatively significantly slow.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A biodegradable magnesium alloy comprising:
5 wt% or less (more than 0) of zinc;
0.35 wt% or less (more than 0) of calcium; and
the balance of magnesium and inevitable impurities,
wherein a microstructure of the magnesium alloy comprises an α-Mg phase matrix and a zinc compound phase which is dispersed and precipitated in the form of particles in the matrix, and
the zinc compound phase comprises 90 wt% or more of Ca₂Mg₆Zn₃ on the basis of the total weight of the zinc compound phase.

2. A biodegradable magnesium alloy comprising:
x wt% of calcium;
y wt% of zinc; and
the balance of magnesium and inevitable impurities,
wherein x and y have a range corresponding to a region in which the lower part of the trajectory of mathematical formula 1 (y = 44.894x² - 25.123x + 5.192) and the upper part of the trajectory of mathematical formula 2 (y = -10.618x² + 7.8784x + 0.1637) overlap in the x-y plane, and
a zinc compound phase comprises 90 wt% or more of Ca₂Mg₆Zn₃ on the basis of the total weight of the zinc compound phase.

3. A biodegradable magnesium alloy comprising:
5 wt% or less (more than 0) of zinc;
x wt% or less (more than 0) of calcium; and
the balance of magnesium and inevitable impurities,
wherein x is a maximum allowable amount of calcium permitting the existence of a temperature section in which a structure formed only of an α-Mg single-phase is phase-transformed into a structure formed of an α-Mg phase matrix and particles, which are dispersed and precipitated in the matrix and formed only of a Ca₂Mg₆Zn₃ phase, in a process of naturally cooling the molten biodegradable magnesium alloy.

4. A biodegradable magnesium alloy comprising:
5 wt% or less (more than 0) of zinc; and
the balance of magnesium and inevitable impurities,
wherein a microstructure of the magnesium alloy comprises an α-Mg phase matrix and a zinc compound phase which is dispersed and precipitated in the form of particles in the matrix.

5. The biodegradable magnesium alloy of claim 1, wherein the microstructure of the magnesium alloy is formed only of both an α-Mg phase matrix and a Ca₂Mg₆Zn₃ phase which is dispersed and precipitated in the matrix.

6. The biodegradable magnesium alloy of claim 1, wherein the calcium is contained in an amount of 0.05-0.35 wt% in the alloy.

7. A manufacturing method for a biodegradable magnesium alloy, the method comprising:
casting a molten metal comprising a composition of the magnesium alloy according to any of claims 1 to 4;
performing solution treatment of the cast magnesium alloy in a temperature range of 300-400°C; and
performing hot extrusion in a temperature range of 300-400°C after performing the solution treatment.

8. The manufacturing method of claim 7, the method further comprising performing artificial aging treatment for 17-19 hours after performing the hot extrusion.

9. An implant comprising the biodegradable magnesium alloy according to any of claims 1 to 4, and being used for orthopedic surgery, dentistry, plastic surgery, or vascular surgery.
